# EUROPEAN PATENT APPLICATION

(11) **EP 1 659 143 A1**
(43) Date of publication of application: **24.05.2006**
(21) Application number: 04748165.0
(22) Date of filing: 27.07.2004
(51) Int. Cl.: C08G 81/00, C08L 71/00, A61F 2/30, A61L 27/00, A61K 47/00

(54) **TEMPERATURE-RESPONSIVE HYDROGEL**

(30) Priority: 28.07.2003 JP 2003280757
(71) Applicant: TEIJIN LIMITED, Osaka-shi Osaka 541-0054 (JP)
(72) Inventor: KITAZONO, Eiichi, c/o Teijin Limited, Hino-shi, Tokyo 191-0065 (JP); KANEKO, Hiroaki, c/o Teijin Limited, Hino-shi, Tokyo 191-0065 (JP)
(74) Representative: Hallybone, Huw George
(86) International application number: PCT/JP2004/011006
(87) International publication number: WO 2005/010080

(57) **Abstract**

A compound made of a hyaluronic acid having from 300 to 30,000 repeating units and a polyalkylene oxide derivative having a specific structure and a specific molecular weight, wherein the content of the polyalkylene oxide derivative is from 5 to 100 equivalents per 100 equivalents of the carboxyl group of the hyaluronic acid; and a hydrogel containing the subject compound.

## Description

### TECHNICAL FIELD

The present invention relates to a compound comprising a hyaluronic acid and a polyalkylene oxide derivative. In more detail, the invention relates to a temperature-responsive hydrogel comprising a hyaluronic acid and a polyalkylene oxide derivative.

### BACKGROUND ART

In recent years, as one of therapeutic methods of largely damaged or lost biotissues and organs, studies of regenerative medicine which is a technology for reconstruction into original biotissues and organs by utilizing differentiation and propagation abilities of cells have become active. Cartilage regeneration is one of them and is positively reviewed as described hereunder.
(1) Cartilage regeneration by using, as a scaffold, a substrate using collagen *(Biomaterials,* **17,** 155-162 (1996))
(2) Cell culture substrate using an insoluble benzyl-esterified hyaluronic acid (U.S. Patent No. 5,939,323; J. *Biomed. Mater. Res.,* **42:2,** 172-81 (1998); *J. Biomed. Mater.* Res., **46:3,** 337-346 (1999); and J. *Ortho. Res.,* **18:5,** 773-380 (2000))
(3) Cartilage cell culture substrate using a crosslinked hyaluronic acid derivative (J. *Ortho. Res.,* **17,** 205-213 (1999))
(4) Tissue regeneration substrate using a polylactic acid or a polyglycolic acid (JP-T-10-513386)

However, in the foregoing examples, a surgical operation must be carried out twice in extracting cells and implanting into a body, and a burden to a patient is very large. In order to solve this problem, it is thought that an endoscopic operation will increase from now on, and development of an artificial material suited for the endoscopic operation will become very important. As required characteristics for artificial materials, it is thought that 1) the shape can be freely controlled (the material can be directly injected into a wound area); and that 2) a cell or a growth factor can be easily embedded. Since a temperature-responsive hydrogel is a very suitable material under this condition, it is thought that merits are large in the regenerative medicine.

The "temperature-responsive hydrogel" as referred to herein can be classified into a low critical solution temperature (LCST) type in which under water circumstances, it hydrates at a temperature lower than a certain temperature and dehydrates at a temperature higher than a certain temperature, thereby causing a volume change; and an upper critical solution temperature (UCST) type in which it reversely hydrates at a temperature higher than a certain temperature, thereby causing a volume change. Of these two types, the hydrogel of a type having properties of LCST such as excellent fastness of response is preferably used in the drug delivery system. The hydrogel of a type having properties of LCST is, for example, uniformly dissolved in an aqueous solution because a mutual action between a polymer and water is preferential at a temperature lower than a certain temperature. However, since when the temperature exceeds a certain temperature, coagulation of a polymer becomes predominant rather than hydration, this hydrogel is a polymer in which dehydration is caused and its aqueous solution becomes cloudy, resulting in precipitation. That is, it is possible to obtain a temperature-responsive hydrogel by using, as the major component, a polymer having properties of LCST in a water-polymer system and subjecting the subject polymer to three-dimensional crosslinking by some method.

As the polymer having properties of LCST in a water-polymer system, there are known N-substituted (meth)acrylamide derivatives such as poly(N-isopropylacrylamide), nitrogen-containing cyclic polymers such as poly(N-acryloylpyrrolidine) and poly(N-acryloylpiperidine), vinyl group-containing amino acids and esters thereof such as poly(N-acryloyl-L-proline), poly(vinylmethyl ether), poly-(ethylene glycol)/poly(propylene glycol), and a polylactic acid-polyglycolic acid-polyethylene oxide copolymer. Of these polymers, a poly(N-isopropylacrylamide) copolymer is representative as a polymer whose transition is sharp and whose phase transition temperature is suitable for application to a biosystem, and studies are being keenly developed from the respective viewpoints of control of the phase transition temperature by the copolymerization component, improvement in the phase transition temperature and elucidation of the phase transition mechanism.

However, in the existing circumstances, there are scarcely temperature-responsive hydrogels exhibiting bioabsorption properties such that they can be implanted into a living body, and only poly(ethylene glycol)/poly(propylene glycol) (*TISSUE ENGINNERING,* Vol. 8, No. 4, 709 (2002)) and a polylactic acid-polyglycolic acid-polyethylene oxide copolymer *(Journal of Controlled* Release, 72, 203 (2001)) are an existing temperature-responsive hydrogel. However, since these polymers are a synthetic polymer, there are considered problems such as low bioaffinity as compared with biomatrix materials. Then, it is prospected that if temperature responsibility can be imparted to a biomatrix material, an ideal temperature-responsive hydrogel having excellent bioabsoprtion properties and bioaffinity is obtained. As an attempt to impart temperature responsibility to a biomatrix material, there are enumerated a chitosan (WO 01/36000) and a hyaluronic acid (WO 99/24070). However, they involve problems such that utilization in a living body is difficult because of a high phase transition temperature and that a phase transition phenomenon cannot be verified in an experiment for corroboration.

### DISCLOSURE OF THE INVENTION

A principal object of the invention is to provide a temperature-responsive hydrogel having excellent bioabsorption properties and bioaffinity. In more detail, the invention is to provide a temperature-responsive hydrogel which can cope with a variety of response temperature regions.

The invention is as follows.
1. A compound comprising a hyaluronic acid and a polyalkylene oxide derivative, represented by the following general formula, wherein the content of the polyalkylene oxide derivative residue is from 5 to 100 equivalents per 100 equivalents of the carboxyl group of the hyaluronic acid: 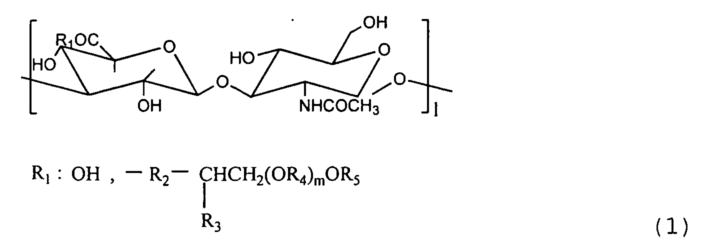 (wherein R₂ represents NH or O; R₃ represents H or CH₃; R₄ represents C₂H₄ or CH₂CH(CH₃); R₅ represents any one of H, CH₃, C₂H₅, and C₄H₉; l represents an integer of from 300 to 30,000; and m represents an integer of from 3 to 140.)

### BRIEF DESCRIPTION OF THE DRAWINGS

Fig. 1 is a phase transition behavior graph of a compound in which JEFFAMINE (registered trademark) XTJ-507 is introduced in an amount of 10 equivalents in terms of hyaluron per 100 equivalents of the carboxyl group of a hyaluronic acid.
Fig. 2 is a phase transition behavior graph of a compound in which JEFFAMINE (registered trademark) XTJ-507 is introduced in an amount of 50 equivalents in terms of hyaluron per 100 equivalents of the carboxyl group of a hyaluronic acid.
Fig. 3 is a phase transition behavior graph of a compound in which JEFFAMINE (registered trademark) XTJ-507 is introduced in an amount of 100 equivalents in terms of hyaluron per 100 equivalents of the carboxyl group of a hyaluronic acid.
Fig. 4 is a phase transition behavior graph of sodium hyaluronate.
Fig. 5 is a phase transition behavior graph of propyl ester hyaluronate.

### BEST MODE FOR CARRYING OUT THE INVENTION

The invention will be hereunder described in detail. Incidentally, these Examples and the like and the description merely exemplify the invention but do not limit the scope of the invention. Needless to say, other embodiments fall with the scope of the invention so far as they coincide with the gist of the invention.

As the hyaluronic acid which is used in the invention, both one which is extracted from animal tissues and one which is produced by a fermentation method can be used. A strain to be used in the fermentation method is a microorganism having a hyaluronic acid producing ability of the *Streptococcus* genus, and examples thereof include *Streptococcus equi* FM-100 (JP-A-63-123392) and *Streptococcus equi* FM-300 (JP-A-2-234689). Materials obtained by cultivation and purification using these variable strains are used. Furthermore, with respect to the molecular weight of the hyaluronic acid, ones having from about 1 × 10⁵ to 1 × 10⁷ daltons are preferable. Incidentally, the hyaluronic acid as referred to the invention also include alkali metal salts thereof, for example, salts of sodium, potassium, and lithium.

As the polyalkylene oxide which is used in the invention, 1) polypropylene glycol or 2) a copolymer comprising poly-(propylene glycol) and poly(ethylene glycol) is preferable. In the case of introduction into the hyaluronic acid by amide bonding, examples include compounds having an amino group in the terminal thereof such as 1-aminopolypropylene glycol methoxide, 1-aminopolypropylene glycol ethoxide, 1-aminopolypropylene glycol propoxide, 1-aminopolypropylene glycol butoxide, 1-aminopoly(propylene glycol)/poly(ethylene glycol) methoxide, 1-aminopoly(propylene glycol)/poly(ethylene glycol) ethoxide, 1-aminopoly(propylene glycol)/poly(ethylene glycol) propoxide, and 1-aminopoly-(propylene glycol)/poly(ethylene glycol) butoxide. Furthermore, in the case of introduction into the hyaluronic acid by ester bonding, examples include compounds having a halogen group in the terminal thereof such as 1-chloropolypropylene glycol methoxide, 1-chloropolypropylene glycol ethoxide, 1-chloropolypropylene glycol propoxide, 1-chloropolypropylene glycol butoxide, 1-chloropoly(propylene glycol)/poly(ethylene glycol) methoxide, 1-chloropoly(propylene glycol)/poly(ethylene glycol) ethoxide, 1-chloropoly(propylene glycol)/poly(ethylene glycol) propoxide, 1-chloropoly(propylene glycol)/poly(ethylene glycol) butoxide, 1-bromopolypropylene methoxide, 1-bromopolypropylene ethoxide, 1-bromopolypropylene propoxide, 1-bromopolypropylene butoxide, 1-bromopoly(propylene glycol)/poly(ethylene glycol) methoxide, 1-bromopoly(propylene glycol)/poly(ethylene glycol) ethoxide, 1-bromopoly(propylene glycol) /poly (ethylene glycol) propoxide, 1-bromopoly(propylene glycol) /poly-(ethylene glycol) butoxide, 1-iodopolypropylene glycol methoxide, 1-iodopolypropylene glycol ethoxide, 1-iodopolypropylene glycol propoxide, 1-iodopolypropylene glycol butoxide, 1-iodopoly(propylene glycol)/poly(ethylene glycol) methoxide, 1-iodopoly(propylene glycol)/poly(ethylene glycol) ethoxide, 1-iodopoly(propylene glycol)/poly(ethylene glycol) propoxide, and 1-iodopoly(propylene glycol)/poly(ethylene glycol) butoxide.

The molecular weight of the foregoing polyalkylene oxide derivative is preferably from 200 to 6, 000. When the molecular weight is not more than 200, a reaction product with the hyaluronic acid does not exhibit temperature responsibility. Also, when the molecular weight is 6, 000 or more, a precipitate is generated so that a hydrogel is not formed.

In the case of using a copolymer comprising poly-(propylene glycol) and poly(ethylene glycol), a copolymerization ratio of poly(propylene glycol) to poly(ethylene glycol) is preferably from 1/99 to 99.9/0.1, and more preferably from 20/80 to 99.9/0.1. When the copolymerization ratio falls outside the foregoing range, a reaction product with the hyaluronic acid does not exhibit temperature responsibility.

The content of the polyalkylene oxide derivative is preferably from 5 to 100 equivalents per 100 equivalents of the carboxyl group of the hyaluronic acid. When the content is not more than 5 equivalents, a reaction product with the hyaluronic acid does not exhibit temperature responsibility.

A typical reaction method between the hyaluronic acid and the polyalkylene oxide derivative includes the following two methods.

### (I) Amide bonding:

Sodium hyaluronate is dissolved in a tetrahydrofuran/water mixed solvent, to which is then added a 1-aminopolyalkylene oxide. 0.1 M HCl and 0.1M NaOH are added to adjust at a pH 6.8, and 1-ethyl-3-[3-(dimethylamino)-propyl]-carbodiimide (EDC) and 1-hydroxybenzotriazole (HOBt) are then added. After stirring overnight, the reaction mixture is purified by dialysis and subjected to freeze-drying to obtain a target compound.

### (II) Ester bonding:

Tetra-n-butylammonium hyaluronate is dissolved in N-methylpyrrolidone, to which is then added a 1-bromopolyalkylene oxide. The mixture is stirred at 37°C for 60 minutes, to which is then added sodium chloride, followed by allowing to stand for 30 minutes. Thereafter, the reaction mixture is reprecipitated with acetone to obtain a target compound.

### [Examples]

The invention will be more specifically described below with reference to the following Examples, but it should not be construed that the invention is limited to these Examples.

Sodium hyaluronate as used in the Examples is sodium hyaluronate having an average molecular weight of 1, 000, 000, which is derived from the *Streptococcus* genus, and is corresponding to one with l = 3,500. With respect to other reagents, 0.1 M HCl, 0.1 M NaOH, 1-ethyl-3- [3- (dimethylamino) - propyl]-carbodiimide (EDC), 1-hydroxybenzotriazole (HOBt), tetra-n-butylammonium bromide, propyl iodide, and N-methylpyrrolidone, all of which are manufactured by Wako Pure Chemical Industries, Ltd., and JEFFAMINE (registered trademark) XTJ-507 (copolymerization ratio of poly(propylene glycol) to poly(ethylene glycol) = 39/6, molecular weight = about 2,000) which is manufactured, by Huntsman Corporation were used.

### [Example 1]

100 mg of sodium hyaluronate was dissolved in 40 mL of tetrahydrofuran/water = 3/2 (v/v). To this solution, JEFFAMINE (registered trademark) XTJ-507 was added in an amount of 120 mg (0.00006 moles) (10 equivalents per 100 equivalents of the carboxyl group of the hyaluronic acid), and 0.1 M HCl and 0.1M NaOH were further added to adjust at a pH 6.8. 12mg (0.000066moles) of 1-ethyl-3-[3-(dimethylamino)-propyl]-carbodiimide (EDC) and 10 mg (0.000066 moles) of 1-hydroxybenzotriazole (HOBt) were dissolved in 10 mL of tetrahydrofuran/water = 3/2, the solution was added in the reaction system, and the mixture was stirred overnight. After stirring, the reaction mixture was purified by dialysis and subjected to free-drying to obtain a target compound. Verification was carried out by ¹H-NMR (JNM-alpha 400, manufactured by JEOL Ltd.), thereby verifying the formation of the target compound.

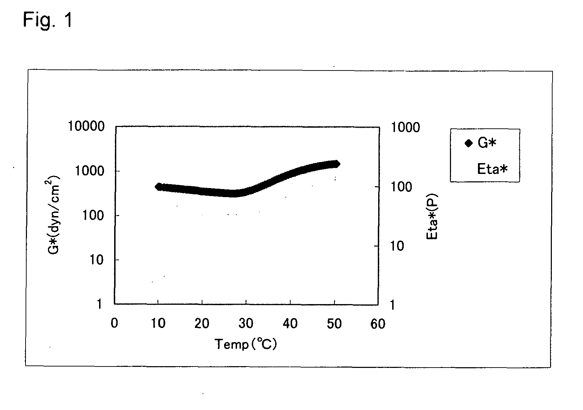

30 mg of the freeze-dried product was dissolved in 970 mg of ion-exchanged water to prepare a hydrogel having a concentration of 3 wt%. In order to examine the phase transition behavior of this hydrogel, complex modulus of elasticity and viscosity in a temperature region from 10 to 50°C were measured by using a rheometer RF III (manufactured by TA Instrument). The results obtained are shown in Fig. 1 (G represents a complex modulus of elasticity, and Eta represents a viscosity). Rises in the complex modulus of elasticity and the viscosity were verified from 30°C, and the complex modulus of elasticity and the viscosity became saturated at 50°C (namely, this means the transition from a sol to a gel). In other words, it has become clear that the temperature phase transition occurred at a temperature between 30 and 50°C.

### [Example 2]

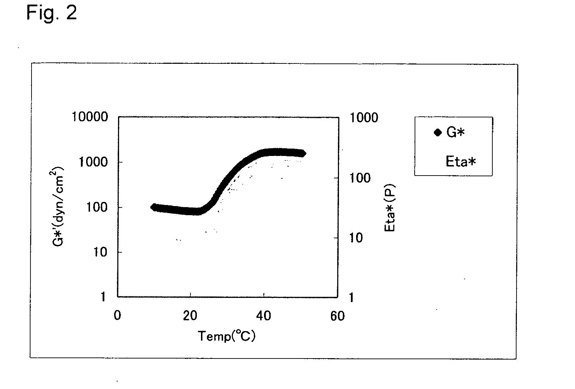

The same procedures as in Example 1 were followed, except for using 600 mg (0.0003 moles) (50 equivalents per 100 equivalents of the carboxyl group of the hyaluronic acid) of JEFFAMINE (registered trademark) XTJ-507, 60 mg (0.00033 moles) of 1-ethyl-3-[3-(dimethylamino)propyl]-carbodiimide (EDC) and 50 mg (0.00033 moles) of 1-hydroxybenzotriazole (HOBt) and changing the concentration to 1 wt%. The results obtained are shown in Fig. 2.

### [Example 3]

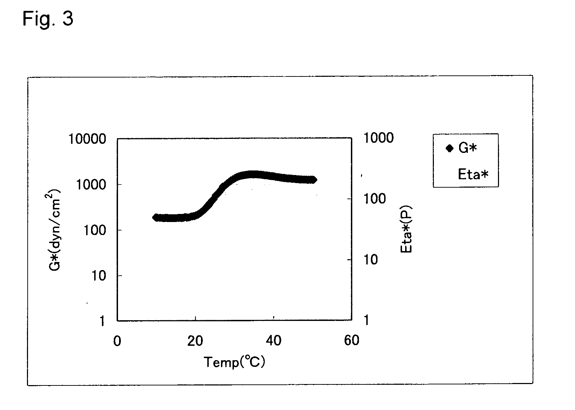

The same procedures as in Example 1 were followed, except for using 1,200 mg (0.0006 moles) (100 equivalents per 100 equivalents of the carboxyl group of the hyaluronic acid) of JEFFAMINE (registered trademark) XTJ-507, 120 mg (0.00066 moles) of 1-ethyl-3-[3-(dimethylamino)propyl]-carbodiimide (EDC) and 100 mg (0.00066 moles) of 1-hydroxybenzotriazole (HOBt) and changing the concentration to 0.5 wt%. The results obtained are shown in Fig. 3.

With respect to the temperature control of phase transition, in comparison of the temperature at which each of the curves of Figs. 1 to 3 corresponding to Examples 1 to 3 rises up, namely the start-up temperature of phase transition, it is noted that when the amount of JEFFAMINE (polyalkylene oxide derivative) is high, the start-up temperature of phase transition is shifted to a lower-temperature side. In other words, by controlling the amount of introduction of JEFFAMINE (polyalkylene oxide derivative), it becomes possible to prepare a hyaluronic acid hydrogel having a desired phase transition temperature.

Besides, it is thought that the phase transition temperature can be changed by the molecular weight of the polyalkylene oxide derivative to be used or the molecular weight of the hyaluronic acid.

In the regenerative medicine region, it is expected that such a hydrogel is applied as an injectable gel for an endoscopic operation. The injectable gel is a liquid in a temperature region lower than the body temperature so that cells or liquid factors can be simply incorporated thereinto, and when injected into a living body, it becomes a gel due to the body temperature, and therefore, it is expected as a scaffold having excellent handling properties. For that reason, any gel cannot be used as an injectable gel unless it causes phase transition in the vicinity of the body temperature.

However, the hydrogel of the invention can be used as an injectable gel because it causes phase transition in the vicinity of the body temperature.

In the light of the above, the invention is able to provide a temperature-responsive hydrogel having excellent bioabsorption properties and bioaffinity, which comprises a hyaluronic acid and a polyalkylene oxide derivative. This temperature-responsive hydrogel is useful as an artificial material in the regenerative medicine while targeting an endoscopic operation.

### [Comparative Example 1]

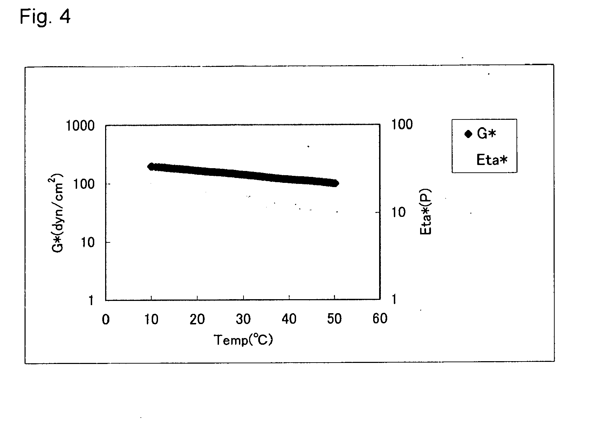

10 mg of sodium hyaluronate was dissolved in 1 mL of water, and the phase transition behavior was observed in the same manner as in Example 1. The results obtained are shown in Fig. 4.

### [Comparative Example 2]

An experiment for corroboration was carried out while referring to WO 99/247070. Details are as follows.

A column (φ1.2 × L20 cm) was charged with an ion exchange resin (DOWEX (registered trademark) 50WX8, total exchange capacity = 1.9 eq/L), and a tetra-n-butylammonium bromide aqueous solution (48 g/100 mL) was flown for displacement. After the displacement, ion-exchanged water was flown until the pH became neutral. Next, a sodium hyaluronate aqueous solution (2 g/1,000 mL) was flown through the column, and freeze-drying was then performed to obtain tetra-n-butylammonium hyaluronate.

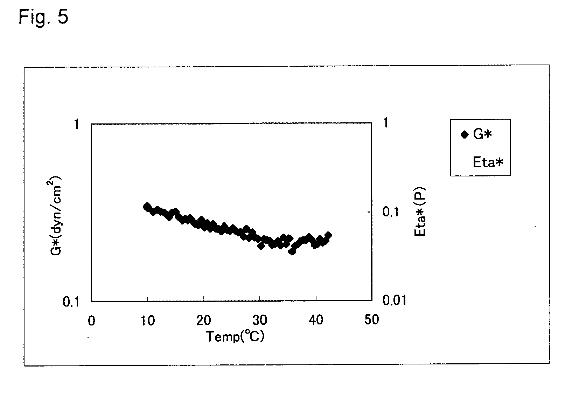

1 g of the resulting tetra-n-butylammonium hyaluronate was dissolved in 50 mL of N-methylpyrrolidone, to which was then gradually added dropwise 0.20 g (0.0012 moles) of propyl iodide at room temperature, and the mixture was stirred at 37°C for 60 hours. After stirring, 1 g of sodium chloride was added, and the mixture was allowed to stand for 30 minutes. Thereafter, 250 mL of acetone was added to obtain a precipitate. The resulting precipitate was rinsed with 200 mL of acetone/water = 80/20 (mL/mL) and dried in vacuo to obtain a target compound. (At this time, silver nitrate is added to verify the elimination of a chloride ion.) Verification was carried out by ¹H-NMR (JNM-alpha 400, manufactured by JEOL Ltd.), thereby verifying the formation (degree of esterification = 50 %) of the target compound. With respect to the phase transition behavior, the observation was carried out under in the same manner as in Example 1 under a condition in a concentration of 15 wt%. The results obtained are shown in Fig. 5.

In all of the hydrogels of Comparative Examples 1 and 2, the phase transition was not verified. According to this fact, the temperature-responsive hydrogel with excellent bioabsorption properties and bioaffinity as obtained by the invention can also be expected to be utilized as an artificial material in the regenerative medicine while targeting an endoscopic operation.

### INDUSTRIAL APPLICABILITY OF THE INVENTION

This temperature-responsive hydrogel is useful as an artificial material in the regenerative medicine while targeting an endoscopic operation.

## Claims

1. A compound comprising a hyaluronic acid and a polyalkylene oxide derivative, represented by the following general formula (1), wherein the content of the polyalkylene oxide derivative residue in R₁ is from 5 to 100 equivalents per 100 equivalents of the carboxyl group of the hyaluronic acid: 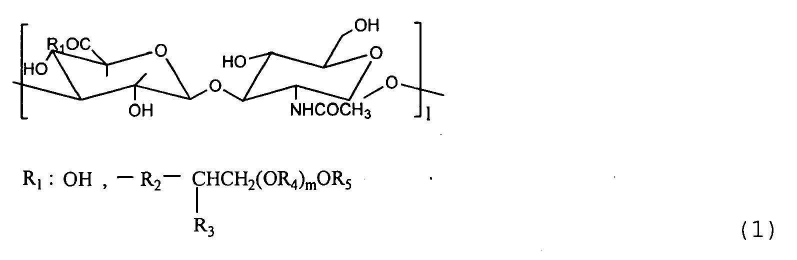 (wherein R₂ represents NH or O; R₃ represents H or CH₃; R₄ represents C₂H₄ or CH₂CH(CH₃); R₅ represents any one of H, CH₃, C₂H₅, and C₄H₉; l represents an integer of from 300 to 30,000; and m represents an integer of from 3 to 140.)

2. A hydrogel comprising the compound according to claim 1.
